# EUROPÄISCHE PATENTSCHRIFT

(11) **EP 1 432 686 B1**
(45) Veröffentlichungstag und Bekanntmachung des Hinweises auf die Patenterteilung: **29.06.2005**
(21) Anmeldenummer: 02767480.3
(22) Anmeldetag: 11.09.2002
(51) Int. Cl.: C07D 221/26, C07D 401/12, A61K 31/47, A61K 31/495, A61P 25/04, A61P 29/00

(54) **TETRAHYDROISOCHINOLINE, IHRE HERSTELLUNG UND VERWENDUNG ALS SCHMERZMITTEL**
TETRAHYDROISOCHINOLINES, THEIR PRODUCTION AND THE USE THEREOF AS ANALGESICS
TETRAHYDROISOCHINOLINES, LEUR PREPARATION ET LEUR UTILISATION EN TANT QU'ANALGESIQUES

(30) Priorität: 24.09.2001 DE 10146867
(43) Veröffentlichungstag der Anmeldung: 30.06.2004
(73) Patentinhaber: Bayer HealthCare AG, 51368 Leverkusen (DE)
(72) Erfinder: GRIEBENOW, Nils, 41541 Dormagen (DE); KALTHOF, Bernd, 42115 Wuppertal (DE); MEIER, Heinrich, 42115 Wuppertal (DE); WIRTZ, Stephan-Nicholas, 42105 Wuppertal (DE); SPREYER, Peter, 42477 Radevormwald (DE)
(86) Internationale Anmeldenummer: PCT/EP2002/010159
(87) Internationale Veröffentlichungsnummer: WO 2003/029221

(56) Entgegenhaltungen:
- EP-A- 0 477 049
- WO-A-96/32382
- US-A- 5 874 443

## Beschreibung

Die Erfindung betrifft neue Tetrahydroisochinoline, Verfahren zu ihrer Herstellung sowie ihre Verwendung zur Herstellung von Arzneimitteln zur Behandlung und/oder Prophylaxe von Krankheiten, insbesondere von Schmerzzuständen.

Neurotensin ist ein biologisch aktives Peptid von 13 Aminosäuren Länge. Entsprechend seiner dualen Funktion als Neurotransmitter und Neuromodulator findet man Neurotensin sowohl im Zentralen Nervensystem als auch in peripheren Geweben. Die biologische Aktivität von Neurotensin wird über Neurotensinrezeptoren auf der Zelloberfläche der Zielgewebe vermittelt. Derzeit sind drei Rezeptoren (NT-1, NT-2 und NT-3) bekannt, die sich in ihrer molekularen Struktur und ihren pharmakologischen Eigenschaften unterscheiden (Vincent et al., *TiPS* 1999, *20*, 302-309).

Der Neurotensin-Rezeptor NT-2 ist ein G-Protein-gekoppelter Rezeptor und wird vorwiegend im Gehirn exprimiert (Vita et al., *Eur. J. Pharmacol.* **1998**, *360,* 265-272). Die Signaltransduktion erfolgt über Inositoltriphosphat-vermittelte Calziumfreisetzung.

Während Neurotensin auf den humanen NT-1 Rezeptor eine agonistische Wirkung ausübt, hat Neurotensin einen antagonistischen Effekt auf den humanen NT-2 Rezeptor.

Die zentrale Applikation von Neurotensin zeigt an Mäusen und Ratten *in vivo* eine Reihe von pharmakologischen Effekten, wie Appetitverminderung (Stanley et al., *Peptides* **1983**, *4*, 493-500), Hypothermie und Analgesie (Tyler et al., *Brain Res.* **1998**, *792*, 246-252).

Während die Appetit unterdrückenden und Temperatur senkenden Eigenschaften des Neurotensins wahrscheinlich über den NT-1 Rezeptor oder bisher noch unbekannte Neurotensinrezeptoren vermittelt sind, wurde mit Hilfe von Antisense Experimenten gezeigt, dass die analgetische Wirkung von Neurotensin mit einiger Sicherheit durch den NT-2 Rezeptor vermittelt wird (Dubuc et al., *J. Neurosci*. **1999**, *19*, 503-510). NT2-Antagonisten sollten daher für die Behandlung von Schmerzzuständen geeignet sein. WO9632382 und EP0477049 offenbaren Pyrazolcarboxamide als Modulatoren von Neurotensinrezeptoren.

Das US-A-5,874,443, die WO 97/16428 und die WO 00/50406 beschreiben Tetrahydroisochinoline enthaltende, chemische Bibliotheken zum Auffinden therapeutisch wirksamer Verbindungen.

Die vorliegende Erfindung betrifft Verbindungen der allgemeinen Formel (I), 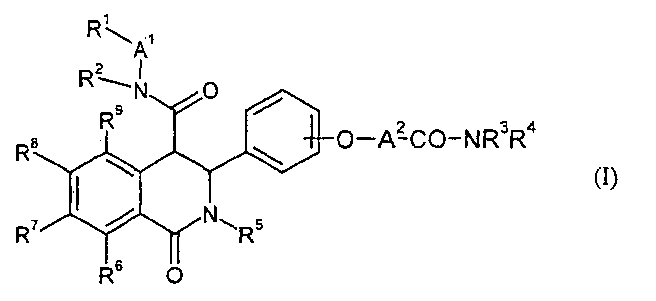 in welcher
- R¹: für Phenyl oder 5- bis 6-gliedriges Heteroaryl steht, wobei gegebenenfalls Phenyl und Heteroaryl gleich oder verschieden durch Reste ausgewählt aus der Gruppe Halogen, Formyl, Carbamoyl, Cyan, Hydroxy, Trifluormethyl, Trifluormethoxy, Nitro, (C₁-C₆)-Alkyl, (C₁-C₆)-Alkoxy und (C₁-C₆)-Alkylthio substituiert sind,
- A¹: für eine Bindung oder (C₁-C₆)-Alkandiyl steht,
- R²: für Wasserstoff oder (C₁-C₆)-Alkyl steht,
- R³ und R⁴: gleich oder verschieden sind und für Wasserstoff, (C₁-C₈)-Alkyl oder (C₃-C₈)-Cycloalkyl stehen,
oder
- R³ und R⁴: gemeinsam mit dem benachbarten Stickstoffatom für einen Rest ausgewählt aus der Gruppe Pyrrolidyl, Piperidyl, Azepinyl und Piperazinyl stehen, wobei die Reste gegebenenfalls durch Methyl oder Ethyl substiuiert sind,
- A²: für (C₁-C₆)-Alkandiyl steht,
- R⁵: für (C₁-C₈)-Alkyl, das gegebenenfalls durch einen Rest ausgewählt aus der Gruppe Trifluormethyl, Halogen, gegebenenfalls teilweise ungesättigtes (C₃-C₈)-Cycloalkyl und (C₆-C₁₀)-Aryl substituiert ist, steht, wobei Aryl seinerseits gegebenenfalls gleich oder verschieden durch Reste ausgewählt aus der Gruppe Halogen, Formyl, Carbamoyl, Cyan, Hydroxy, Trifluormethyl, Trifluormethoxy, Nitro, (C₁-C₆)-Alkyl, (C₁-C₆)-Alkoxy und (C₁-C₆)-Alkylthio substituiert ist,
und
- R⁶, R⁷, R⁸ und R⁹: gleich oder verschieden sind und für Wasserstoff, Halogen, Formyl, Carbamoyl, Cyan, Hydroxy, Trifluormethyl, Trifluormethoxy, Nitro, (C₁-C₆)-Alkyl, (C₁-C₆)-Alkoxy oder (C₁-C₆)-Alkylthio stehen,
und deren Salze, Hydrate und/oder Solvate.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Hydrate und/oder Solvate vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Hydrate der erfindungsgemäßen Verbindungen sind stöchiometrische Zusammensetzungen der Verbindungen oder ihrer Salze mit Wasser.

Solvate der erfindungsgemäßen Verbindungen sind stöchiometrische Zusammensetzungen der Verbindungen oder ihrer Salze mit Lösungsmittel.

Im Rahmen der vorliegenden Erfindung haben die Substituenten im Allgemeinen die folgende Bedeutung:
(C₁-C₆)-Alkandiyl steht für einen geradkettigen oder verzweigten Alkandiylrest mit 1 bis 6 Kohlenstoffatomen. Bevorzugt ist ein geradkettiger oder verzweigter Alkandiylrest mit 1 bis 4 Kohlenstoffatomen. Beispielsweise und vorzugsweise seien genannt Methylen, Ethylen, Propylen, Propan-1,2-diyl, Propan-2,2-diyl, Butan-1,3-diyl, Butan-2,4-diyl, Pentan-2,4-diyl, 2-Methyl-pentan-2,4-diyl.
   Wenn eine Methylengruppe des Alkandiyl-Rests gegebenenfalls durch ein Sauerstoff- oder Schwefelatom ersetzt ist, seien beispielsweise und vorzugsweise genannt: -O-, -S-, -O-CH₂-, -S-CH₂-, -CH₂-O-, -CH₂-S-, -CH₂-O-CH₂-, -O-CH₂-CH₂-, 1-Oxa-propan-1,2-diyl, 3-Oxa-butan-2,4-diyl, 3-Thia-butan-2,4-diyl.
(C₁-C₆)-Alkoxy steht für einen geradkettigen oder verzweigten Alkoxyrest mit 1 bis 6 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.Butoxy, n-Pentoxy und n-Hexoxy.
(C₁-C₈)- und (C₁-C₆)-Alkyl stehen für einen geradkettigen oder verzweigten Alkylrest mit 1 bis 8 bzw. 1 bis 6 Kohlenstoffatomen. Bevorzugt ist ein geradkettiger oder verzweigter Alkylrest mit 1 bis 4, besonders bevorzugt mit 1 bis 3 Kohlenstoffatomen. Beispielsweise und vorzugsweise seien genannt: Methyl, Ethyl, n-Propyl, Isopropyl, tert.Butyl, n-Pentyl, n-Hexyl, n-Heptyl und n-Octyl.
(C₁-C₆)-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.Butylthio, n-Pentylthio und n-Hexylthio.
(C₃-C₈)-Cycloalkyl steht für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.
   Teilweise ungesättigte Cycloalkylreste sind nicht-aromatische Cycloalkylreste, die eine oder mehrere Mehrfachbindungen, vorzugsweise Doppelbindungen enthalten. Beispielsweise und vorzugsweise seien genannt: Cyclopentenyl, Cyclohexenyl und Cycloheptenyl.
Halogen steht für Fluor, Chlor, Brom und Jod. Bevorzugt sind Fluor, Chlor und Brom. Besonders bevorzugt sind Fluor und Chlor.
5- bis 6-gliedriges Heteroaryl steht für einen aromatischen Rest mit 5 bis 6 Ringatomen und bis zu 4 Heteroatomen aus der Reihe S, O und/oder N. Der Heteroarylrest kann über ein Kohlenstoff- oder Heteroatom gebunden sein. Beispielsweise und vorzugsweise seien genannt: Thienyl, Furyl, Pyrrolyl, Thiazölyl, Oxazolyl, Imidazolyl, Tetrazolyl, Pyridyl, Pyrimidinyl, und Pyridazinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der allgemeinen Formel (I), in welchen die Substituenten in den Positionen 3 und 4 am 1,2,3,4-Tetrahydroisochinolin trans zueinander stehen. Die relative trans-Konfiguration der erfindungsgemäßen Verbindungen kann beispielhaft durch die allgemeine Formel (Ia) veranschaulicht werden:

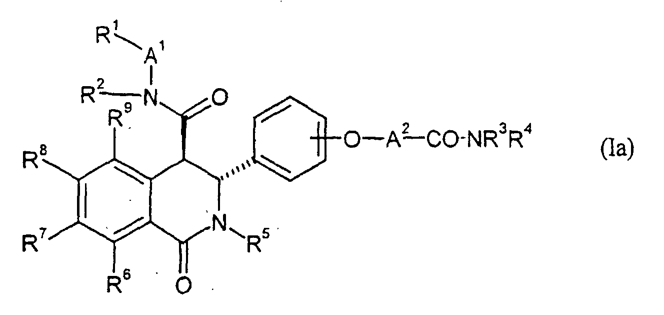

Außerdem sind Verbindungen der allgemeinen Formel (Ib) bevorzugt, 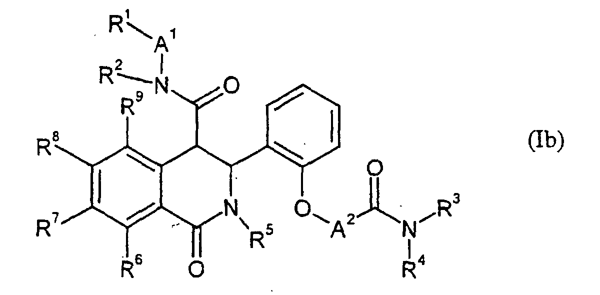 in welchen R¹, A¹, R², R³, R⁴, A², R⁵, R⁶, R⁷, R⁸ und R⁹ die oben angegebene Bedeutung haben.

Ebenso bevorzugt sind Verbindungen der allgemeinen Formel (I), in welchen R¹ für 3-Pyridyl steht und A¹, R², R³, R⁴, A², R⁵, R⁶, R⁷, R⁸ und R⁹ die oben angegebene Bedeutung haben.

Ebenso bevorzugt sind Verbindungen der allgemeinen Formel (I), in welchen A¹ für Methylen steht und R¹, R², R³, R⁴, A², R⁵, R⁶, R⁷, R⁸ und R⁹ die oben angegebene Bedeutung haben.

Ebenso bevorzugt sind Verbindungen der allgemeinen Formel (I), in welchen R² für Wasserstoff steht und R¹, A¹, R³, R⁴, A², R⁵, R⁶, R⁷, R⁸ und R⁹ die oben angegebene Bedeutung haben.

Ebenso bevorzugt sind Verbindungen der allgemeinen Formel (I), in welchen R³ und R⁴ für Wasserstoff stehen und R¹, A¹, R², A², R⁵, R⁶, R⁷, R⁸ und R⁹ die oben angegebene Bedeutung haben.

Ebenso bevorzugt sind Verbindungen der allgemeinen Formel (I), in welchen A² für Methylen steht und R¹, A¹, R², R³, R⁴, R⁵, R⁶, R⁷, R⁸ und R⁹ die oben angegebene Bedeutung haben.

Ebenso bevorzugt sind Verbindungen der allgemeinen Formel (I), in welchen
- R⁵: für (C₁-C₃)-Alkyl, das durch einen Rest ausgewählt aus der Gruppe Trifluormethyl, gegebenenfalls einfach ungesättigtes (C₅-C₇)-Cycloalkyl und gegebenenfalls durch Methyl, Halogen oder Methoxy substituiertes Phenyl substituiert ist, steht, oder
für (C₄-C₆)-Alkyl, das gegebenenfalls durch Trifluormethyl substituiert ist, steht,
und R¹, A¹, R², R³, R⁴, A², R⁶, R⁷, R⁸ und R⁹ die oben angegebene Bedeutung haben.

Ebenso bevorzugt sind Verbindungen der allgemeinen Formel (I), in welchen R⁶, R⁷, R⁸ und R⁹ gleich oder verschieden sind und für Wasserstoff, Methyl, Fluor, Chlor oder Methoxy, besonders bevorzugt für Wasserstoff stehen.

Besonders bevorzugt sind Kombinationen von zwei oder mehreren der oben genannten Vorzugsbereiche.

Ganz besonders bevorzugt sind Verbindungen der allgemeinen Formel (I), in welchen
- R¹: für 3-Pyridyl, das gegebenenfalls gleich oder verschieden durch Chlor, Fluor oder Methyl substituiert ist, steht,
- A¹: für Methylen steht,
- R²: für Wasserstoff steht,
- R³ und R⁴: für Wasserstoff stehen,
- A²: für Methylen steht,
- R⁵: für (C₁-C₃)-Alkyl, das durch einen Rest ausgewählt aus der Gruppe Trifluormethyl, gegebenenfalls einfach ungesättigtes (C₅-C₇)-Cycloalkyl und gegebenenfalls durch Methyl, Halogen oder Methoxy substituiertes Phenyl substituiert ist, steht, oder
für (C₄-C₆)-Alkyl, das gegebenenfalls durch Trifluormethyl substituiert ist, steht,
und
R⁶, R⁷, R⁸ und R⁹ für Wasserstoff stehen,
und deren Salze, Hydrate und/oder Solvate.

Die Erfindung betrifft weiterhin ein Verfahren zur Herstellung der Verbindungen der Formel (I), dadurch gekennzeichnet, dass man Verbindungen der allgemeinen Formel (II) 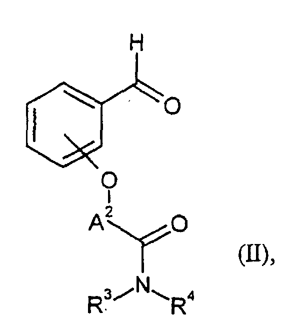 in welcher
A², R³ und R⁴ die oben angegebene Bedeutung haben,
zunächst in Gegenwart wasserentziehender Mittel, wie beispielsweise Natriumsulfat oder Orthoameisensäureestern, vorzugsweise Orthoameisensäuretrimethylester, mit Aminen der allgemeinen Formel (III)

R⁵-NH₂ (III),

in welcher
R⁵ die oben angegebene Bedeutung hat,
zu Verbindungen der allgemeinen Formel (IV) 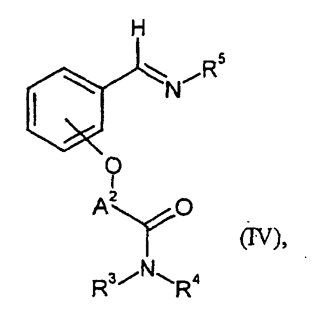 in welcher
A², R³, R⁴ und R⁵ die oben angegebene Bedeutung haben,
umsetzt, diese dann, in situ oder unter vorheriger Isolierung als Zwischenstufe, gegebenenfalls unter Zusatz einer Hilfsbase mit einem Homophthalsäureanhydrid der allgemeinen Formel (V) 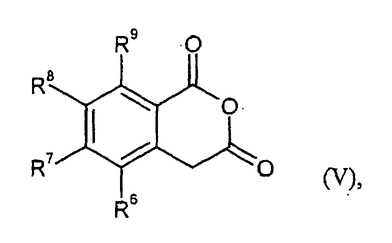 in welcher
R⁶, R⁷, R⁸ und R⁹ die oben angegebene Bedeutung haben,
in Verbindungen der allgemeinen Formel (VI) 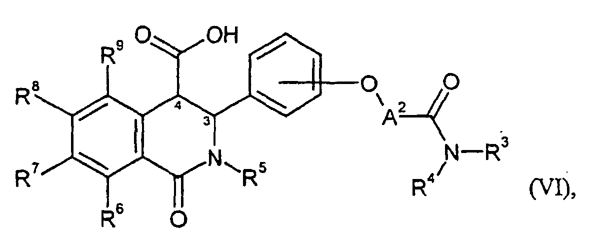 in welcher
A², R³, R⁴, R⁵, R⁶, R⁷, R⁸ und R⁹ die oben angegebene Bedeutung haben,
überführt gegebenenfalls durch Erhitzen in Gegenwart einer Säure zur relativen trans-Konfiguration der Positionen 3 und 4 im Tetrahydroisochinolin-Ring, wie in der allgemeinen Formel (Ia) veranschaulicht, epimerisiert und abschließend unter Aktivierung der Carbonsäure-Gruppe in (VI) mit Verbindungen der allgemeinen Formel (VII)

R¹-A¹-NH-R² (VII),

in welcher
A¹, R¹ und R² die oben angegebene Bedeutung haben,
umsetzt.

Das erfindungsgemäße Verfahren wird im Allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für das Verfahren eignen sich übliche organische Lösemittel, die sich unter den Reaktionsbedingungen nicht verändern. Hierzu gehören Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Ethylacetat, Pyridin, Dimethylsulfoxid, Dimethylformamid, N,N'-Dimethylpropylenharnstoff (DMPU), N-Methylpyrrolidon (NMP), Acetonitril, Aceton oder Nitromethan. Ebenso ist es möglich, Gemische der genannten Lösemittel zu verwenden.

Bevorzugte Lösemittel für die Verfahrensschritte (II) + (III) → (IV) und (IV) + (V) → (VI) sind Dichlormethan, 1,2-Dichlorethan, Toluol oder Gemische dieser Lösemittel. Für den Verfahrensschritt (VI) + (VII) → (I) ist Dichlormethan oder Dimethylformamid bevorzugt.

Die erfindungsgemäßen Verfahrensschritte (II) + (III) → (IV) und (IV) + (V) → (VI) werden im Allgemeinen in einem Temperaturbereich von 0°C bis +60°C, bevorzugt von +20°C bis +40°C, durchgeführt. Der Verfahrensschritt (VI) + (VII) → (I) wird im Allgemeinen in einem Temperaturbereich von -20°C bis +40°C, bevorzugt von 0°C bis +25°C durchgeführt.

Als Hilfsstoffe für die Amidbildung im Verfahrensschritt (VI) + (VII) → (I) werden bevorzugt übliche Kondensationsmittel eingesetzt, wie Carbodiimide, z.B. N,N'-Diethyl-, N,N'-Dipropyl-, N,N'-Diisopropyl-, N,N'-Dicyclohexylcarbodiimid (DCC); N-(3-Dimethylaminoisopropyl)-N'-ethylcarbodiimid-Hydrochlorid (EDCxHCl), oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchlorforrniat, oder Bis-(2-oxo-3-oxazolidinyl)-phosphorylchlorid oder Benzotriazolyloxy-tris(dimethylamino)phosphonium-hexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluronium-tetrafluoroborat (TPTU) oder O-(7-Azabenzotriazol-1-yl)-N,N,N',N'-tetramethyluronium-hexafluorophosphat (HATU), gegebenenfalls in Kombination mit weiteren Hilfsstoffen wie 1-Hydroxybenzotriazol oder N-Hydroxysuccinimid, sowie als Basen Alkalicarbonate, z.B. Natrium- oder Kaliumcarbonat oder -hydrogencarbonat, oder organische Basen wie Trialkylamine, z.B. Triethylamin, N-Methylmorpholin, N-Methylpiperidin oder Diisopropylethylamin. Besonders bevorzugt ist die Kombination von EDC, 1-Hydroxybenzotriazol und N-Methylmorpholin oder Triethylamin.

Als Hilfsbase für die Reaktion (IV) + (V) → (VI) eignen sich die üblichen organischen Basen wie Trialkylamine, z.B. Triethylamin, N-Methylmorpholin, N-Methyl-piperidin oder Diisopropylethylamin. Bevorzugt ist Triethylamin.

Als Säure zur Epimerisierung in die relative trans-Konfiguration der Positionen 3 und 4 in den Verbindungen der allgemeinen Formel (VI) eignen sich im Allgemeinen Trifluoressigsäure, Schwefelsäure, Chlorwasserstoff, Bromwasserstoff und Essigsäure oder deren Gemische gegebenenfalls unter Zusatz von Wasser. Bevorzugt ist Essigsäure. Die Epimerisierung wird im Allgemeinen in einem Temperaturbereich von +20°C bis +150°C, bevorzugt von +80°C bis +120°C durchgeführt.

Die Verbindungen der allgemeinen Formel (II) sind bekannt oder können beispielsweise dadurch hergestellt werden, dass man
[A] Verbindungen der allgemeinen Formel (VIII) 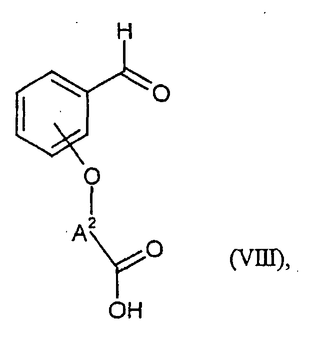 in welcher
   A² die oben angegebene Bedeutung hat,
   unter Aktivierung der Carbonsäure-Gruppe mit Aminen der allgemeinen Formel (IX)

   R³-NH-R⁴ (IX),

   in welcher
   R³ und R⁴ die oben angegebene Bedeutung haben,
   umsetzt, oder
[B] Hydroxybenzaldehyde der allgemeinen Formel (X) 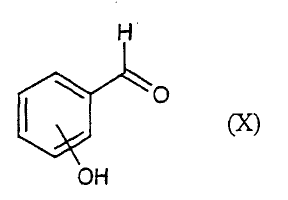 in Gegenwart einer Base mit Verbindungen-der allgemeinen Formel (XI) 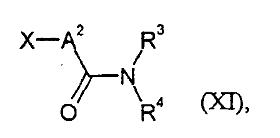 in welcher
   A², R³ und R⁴ die oben angegebene Bedeutung haben,
   und
   - X: für eine geeignete Abgangsgruppe, wie beispielsweise Halogen, Mesylat oder Tosylat, vorzugsweise für Brom oder Iod steht,
umsetzt.

Die Amidbildung (VIII) + (IX) → (II) erfolgt analog zum oben beschriebenen Verfahrensschritt (VI) + (VII) → (I) bevorzugt in Dichlormethan oder Dimethylformamid als Lösemittel unter Verwendung der Kombination EDC, 1-Hydroxybenzotriazol und N-Methylmorpholin oder Triethylamin.

Die Umsetzung (X) + (XI) → (II) wird bevorzugt in Dimethylformamid als Lösemittel im Allgemeinen in einem Temperaturbereich von 0°C bis +120°C, bevorzugt von +60°C bis +80°C durchgeführt.

Als Basen für die Umsetzung (X) + (XI) → (II) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Natrium-, Kalium- oder Calciumcarbonat, Alkali-Alkoholate wie Natrium- oder Kaliummethanolat, Natrium- oder Kaliumethanolat oder Kalium-tert.-butylat, Alkalihydride wie Natriumhydrid, Amide wie Natriumamid, Lithium-bis-(trimethylsilyl)amid oder Lithiumdiisopropylamid, oder organische Amine wie Pyridin, Triethylamin, Ethyldiisopropylamin, N-Methylmorpholin oder N-Methylpiperidin. Bevorzugt ist Kalium-tert.-butylat.

Die Verbindungen der allgemeinen Formeln (III), (V), (VII), (VIII), (IX), (X) und (XI) sind kommerziell erhältlich, bekannt oder können nach bekannten Verfahren hergestellt werden.

Das erfindungsgemäße Verfahren kann durch das folgende Syntheseschema verdeutlicht werden:

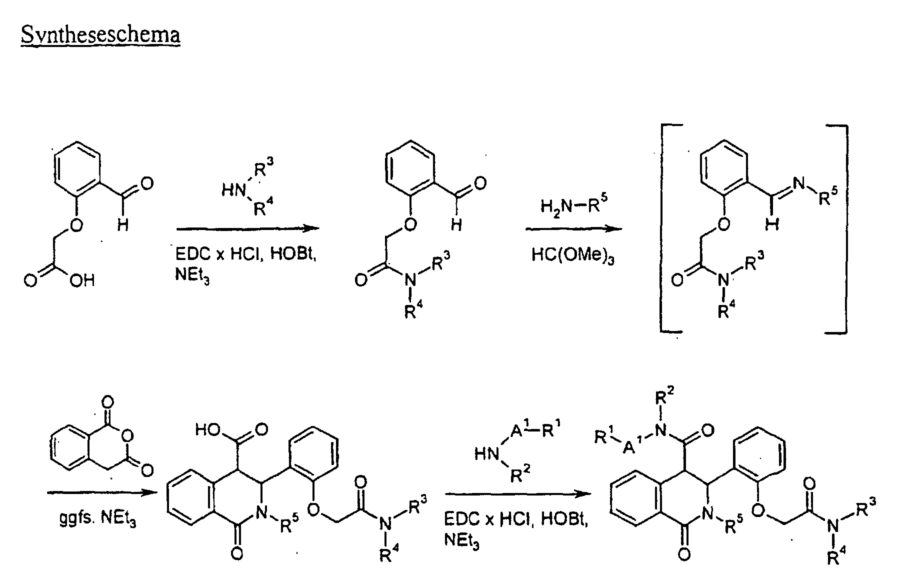

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie wirken antagonistisch am NT2-Rezeptor.

Die erfindungsgemäßen Verbindungen können aufgrund ihrer pharmakologischen Eigenschaften allein oder in Kombination mit anderen Arzneimitteln zur Behandlung und/oder Prävention insbesondere von Schmerzzuständen eingesetzt werden.

Die in vitro-Wirkung der erfindungsgemäßen Verbindungen kann mit folgendem Assay gezeigt werden:

### NT2-Antagonismus-Assay

Die Aktivierung des humanen Neurotensin-2 (NT-2) Rezeptors durch einen Agonisten wie beispielsweise SR48692, 2-{[1-(7-chlor-chinolin-4-yl)-5-(2,6-dimethoxyphenyl)pyrazol-3-yl]carbonylamino}tricyclo[3.3.3.1^{3,7}]decan-2-carbonsäure, führt über Stimulierung der Phospholipase C zur Freisetzung von Kalziumionen aus intrazellulären Speichern. Antagonisten blockieren die Aktivierung des Rezeptors durch den Agonisten und damit auch die Agonisten-abhängige Stimulation der Phospholipase C und die dadurch ausgelöste intrazelluläre Kalziumfreisetzung.

Ein funktioneller *in vitro*-Assay kann mit stabilen Zellenlinien, z.B. CHO oder HEK 293, die den humanen NT-2 Rezeptor rekombinant exprimieren, durchgeführt werden. Dabei wird die Aktivität des Rezeptors über Messung der daran gekoppelten intrazellulären Kalziumfreisetzung bestimmt (in Mikrotiterplatten mit 96, 384 und 1536 Vertiefungen / Platte). Die konzentrationsabhängige Wirkung der getesteten NT-2 Liganden auf die Rezeptoraktivität kann als IC₅₀ angegeben werden.

Das Enantiomer B des Beispiels 1 hat beispielsweise einen IC₅₀-Wert von weniger als 0,2 µM.

Die Eignung der erfindungsgemäßen Verbindungen zur Behandlung von Schmerzzuständen kann in geeigneten Tiermodellen gezeigt werden.

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DCI: direkte chemische Ionisation (bei MS)
- DMF: *N,N*-Dimethylformamid
- DMSO: Dimethylsulfoxid
- ESI: Elektrospray-Ionisation (bei MS)
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- RP: Reverse Phase
- RT: Raumtemperatur
- Rₜ: Retentionszeit (bei HPLC)

### Ausgangsverbindungen und Zwischenprodukte:

### Beispiel I

### 2-(2-Formylphenoxy)acetamid

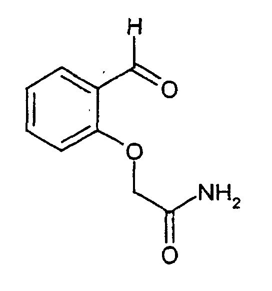

Zu einer Lösung von 13,1 g (107 mmol) Salicylaldehyd in DMF (400 ml) werden 10,9 g (97,2 mmol) Kalium-tert.-butylat gegeben, anschließend wird das Gemisch für 60 min bei 60°C gerührt. Dann tropft man 13.7 g 2-Bromacetamid (98-prozentig, entspricht 97,2 mmol) in DMF (100 ml) hinzu und lässt bei 80°C über Nacht rühren. Nach dem Abkühlen wird das Solvens im Vakuum entfernt und der Rückstand zwischen Essigsäureethylester und Wasser verteilt. Die Phasen werden getrennt und die wässrige Phase dreimal mit Essigsäureethylester extrahiert. Die vereinigten organischen Phasen werden mit 1 N Salzsäure, Wasser, gesättigter Natriumhydrogencarbonatlösung sowie gesättigter Natriumchloridlösung gewaschen und über Magnesiumsulfat getrocknet. Das Solvens wird im Vakuum entfernt und der Rückstand aus Essigsäureethylester umkristallisiert. Man erhält so 13,4 g (77 %) der Zielverbindung als Kristalle mit einem Schmelzpunkt von 127°C.
MS (DCI/NH₃): 197 (M+NH₄)⁺
HPLC: Rₜ = 3.13 min
¹H-NMR (400 MHz, CDCl₃): δ = 4.59 (s, 2H), 5.79 (s, br, 1H), 6.95 (d, 1H), 7.20 (t, 1H), 7.56 (s, br, 1H), 7.61 (td, 1H), 7.79 (dd, 1H), 10.18 (s, 1H).

### Beispiel II

### 2-[2-(4-Methyl-1-piperazinyl)-2-oxoethoxy]benzaldehyd

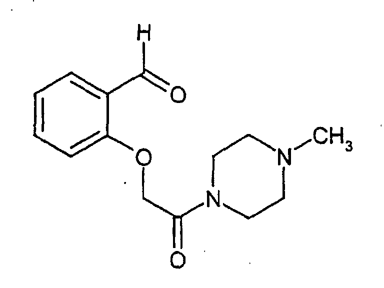

5,00 g (27,8 mmol) 2-(2-Formylphenoxy)essigsäure, 2,78 g (27,8 mmol) 1-Methylpiperazin und 3,75 g (27,8 mmol) 1-Hydroxy-1H-benzotriazol werden in 20 ml Dichlormethan gelöst und nach Zugabe von 5,62 g (55,5 mmol) Triethylamin und 6,38 g (33,3 mmol) N-(3-Dimethylaminopropyl)-N'-ethylcarbodiimid-Hydrochlorid über Nacht bei RT gerührt. Zur Aufarbeitung wird das Reaktionsgemisch mit Dichlormethan verdünnt, zweimal mit Wasser gewaschen, getrocknet und eingeengt. Es verbleiben 8,12 g der Zielverbindung als brauner Feststoff in einer HPLC-Reinheit von 89 % (Ausbeute annähernd quantitativ).
MS (DCI/NH₃): 263 (M+H)⁺
HPLC: Rₜ = 2.93 min
¹H-NMR (300 MHz, DMSO-d₆): δ = 2.1-2.4 (m, 6H), 2.28 (s, 3H), 3.45 (t, 4H), 5.07 (s, 2H), 7.08 (t, 1H), 7.14 (d, 1H), 7.62 (ddd, 1H), 7.70 (dd, 1H), 10.45 (s, 1H).

In analoger Weise wurden erhalten:

### Beispiel III

### 2-[2-Oxo-2-(1-piperidinyl)ethoxy]benzaldehyd

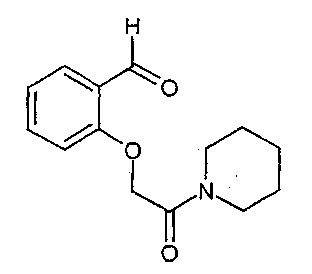

MS (ESIpos): 248 (M+H)⁺
HPLC: Rₜ = 3.87 min
¹H-NMR (200MHz, DMSO-d₆): δ = 1.3-1.7 (m, 6H), 3.3-3.5 (m, 4H), 5.05 (s, 2H), 7.0-7.2 (m, 2H), 7.55-7.75 (m, 2H), 10.46 (s, 1H).

### Beispiel IV

### 2-(2-Formylphenoxy)essigsäureisopropylamid

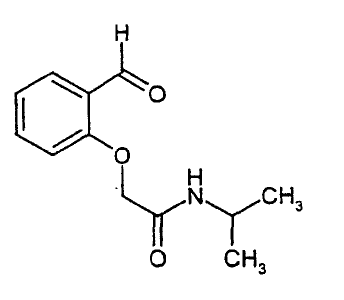

MS (DCI/NH₃): 239 (M+NH₄)⁺
HPLC: Rₜ = 3.63 min
¹H-NMR (300 MHz, DMSO-d₆): δ = 1.10 (d, 6H), 3.96 (m, 1H), 4.63 (s, 2H), 7.08-7.18 (m, 2H), 7.65 (td, 1H), 7.75 (dd, 1H), 8.00 (d, br, 1H), 10.41 (s, 1H).

### Beispiel V

### 3,4-trans-3-[2-(Carbamoylmethoxy)phenyl]-2-[2-(1-cyclohexen-1-yl)ethyl]-1-oxo-1,2,3,4-tetrahydro-4-isochinolincarbonsäure

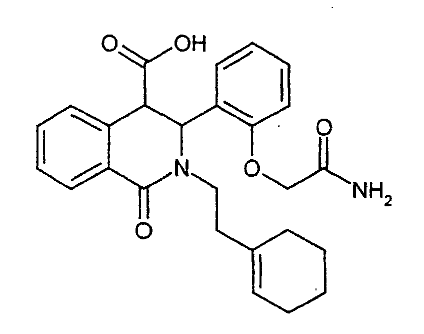

4,99 g (39,9 mmol) 2-(Cyclohex-1-enyl)-ethylamin werden in 40 ml Dichlormethan gelöst und bei RT mit 6,50 g (61,2 mmol) Orthoameisensäuretrimethylester und 6,93 g (38,7 mmol) 2-(2-Formylphenoxy)acetamid versetzt. Die Mischung wird über Nacht gerührt, dann gibt man 5,33 g (32,9 mmol) Homophthalsäureanhydrid hinzu. Das Reaktionsgemisch erwärmt sich leicht und nach kurzer Zeit fällt ein farbloser Feststoff aus. Man lässt 16 h nachrühren, filtriert und wäscht den Rückstand mit wenig Dichlormethan. Das getrocknete Rohprodukt enthält ein cis/trans-Isomerengemisch der angebenen Konstitutionsformel und wird durch sechsstündiges Kochen in Eisessig zu weitgehend einheitlichem trans-Isomer epimerisiert. Nach Abkühlen wird die Essigsäure im Vakuum entfernt, der braune Rückstand in Dichlormethan gelöst und mit Wasser sowie gesättigter Natriumchloridlösung gewaschen. Das Produkt wird an Kieselgel adsorbiert und durch Flash-Chromatographie weiter gereinigt (Dichlormethan/Methanol-Gradient 95:5 - 5:1), anschließend aus Wasser/-Isopropanol umkristallisiert. Es verbleiben 4,28 g (29 %) der Zielverbindung in 96prozentiger Reinheit nach HPLC.
MS (ESIpos): 449 (M+H)⁺
HPLC: Rₜ = 4.19 min
¹H-NMR (300 MHz, DMSO-d₆): δ = 1.4-1.6 (m, 4H), 1.8-2.0 (s, br, 4H), 2.16 (m, 2H), 2.76 (m, 1H), 4.03 (m, 1H), 4.59 (AB-System, 2H), 5.38 (s, br, 1H), 5.68 (s, 1H), 6.56 (d, 1H), 6.76 (t, 1H), 6.97 (d, 1H), 7.1-7.25 (m, 2H), 7.3-7.45 (m, 2H), 7.48 (s, br, 1H), 7.61 (s, br, 1H), 7.88-7.98 (m, 1H), 13.05 (s, br, 1H).

Das racemische Produkt lässt sich durch präparative Hochdruckflüssigkeits-chromatographie an chiralem Trägermaterial in die Enantiomere trennen (Säule: Daicel Chiralpak AS 10 µm, 250 x 20 mm, Eluent: 90 % Acetonitril mit 0,2 % Trifluoressigsäure/10 % Ethanol, Flussrate: 10 ml/min, Injektionsvolumen: 1000 µl, Temperatur: 40°C).
Enantiomer A: Rₜ = 5.14 min; [α]_{D} ^{20.1} (c = 0,55; CHCl₃) = - 23.4
Enantiomer B: Rₜ = 7.01 min; [α]_{D} ^{20.0} (c = 0,55; CHCl₃) = + 27.5
(Säule: Daicel Chiralpak AS 10 µm 250 x 4,6 mm, Eluent: Acetonitril mit 0,2 % Trifluoressigsäure, Flussrate: 1 ml/min, Injektionsvolumen: 3 µl, Temperatur: 40°C).

### Beispiel VI

### 3,4-trans-3-[2-(Carbamoylmethoxy)phenyl]-2-(2-phenylethyl)-1-oxo-1,2,3,4-tetrahydro-4-isochinolincarbonsäure

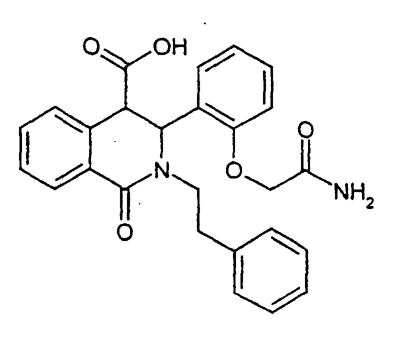

### Erster Reaktionsschritt:

Eine Lösung von 0,75 g (6,17 mmol) 2-Phenylethylamin und 1,11 g (6,17 mmol) 2-(2-Formylphenoxy)essigsäureamid wird in 10 ml Orthoameisensäuretrimethylester über Nacht bei RT gerührt. Der Ansatz wird dreimal im doppelten Volumen Toluol aufgenommen und jeweils im Vakuum eingeengt.

### Zweiter Reaktionsschritt:

Der Rückstand wird in 30 ml Dichlorethan gelöst und mit 1,25 g (12,3 mmol) Triethylamin und 1,00 g (6,17 mmol) Homophthalsäureanhydrid versetzt. Man lässt die Reaktionsmischung über Nacht rühren, verdünnt mit Dichlormethan und extrahiert mit Wasser. Die organische Phase wird mit gesättigter Natriumchloridlösung gewaschen, über Natriumsulfat getrocknet und im Vakuum eingeengt. Der Rückstand wird an einer RP-Säule mit Acetonitril/Wasser (HPLC, 30:70 - 95:5) chromatografiert. Das so als Epimerengemisch erhaltene Rohprodukt wird durch Kochen über Nacht in Eisessig (10 ml) epimerisiert. Die Lösung wird auf 100 ml Wasser gegeben und das Gemisch mit Essigsäureethylester extrahiert. Die organische Phase wird über Natriumsulfat getrocknet und im Vakuum eingeengt. Zur Beseitigung von Säureresten wird mehrfach in Toluol aufgenommen und im Vakuum eingeengt. Der gelbe Rückstand wird in Aceton verrieben, abfiltriert und im Vakuum getrocknet. Man erhält so 527 mg (19 %) der Zielverbindung als farblosen Feststoff. Durch Eindampfen der Mutterlauge gewinnt man eine zweite Fraktion geringerer Reinheit als gelblichen Feststoff: 910 mg, 65%-prozentig nach HPLC.
¹H-NMR (400 MHz, CDCl₃): δ = 2.65-3.05 (m, 4H), 4.05-4.25 (m, 1H), 4.34 (s, 1H), 4.61 (AB-System, 2H), 5.83 (s, 1H), 6.57 (d, 1H), 6.75 (t, 1H), 6.97 (d, 1H), 7.1-7.3 (m, 6H), 7.3-7.58 (m, 2H), 7.55 (s, br, 1H), 7.64 (s, br, 1H), 7.88-8.0 (m, 1H), 13.15 (s, br, 1H).

In analoger Weise wurden die folgenden Verbindungen hergestellt:

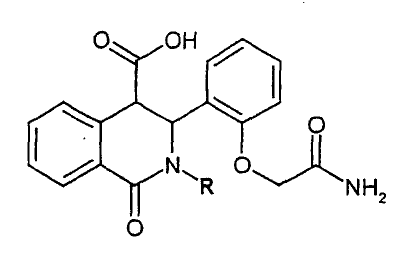

### Beispiel VII

### 3,4-trans-3-[2-(Carbamoylmethoxy)phenyl]-2-[2-(3,4-dimethoxyphenyl)ethyl]-1-oxo-1,2,3,4-tetrahydro-4-isochinolincarbonsäure

Ausgehend von 2-(3,4-Dimethoxyphenyl)ethylamin [R = 2-(3,4-dimethoxyphenyl)-ethyl]:
LC-MS (Methode B): Rₜ = 2.30 min
MS (ESIpos): m/z = 505 (M+H)⁺.

### Beispiel VIII

### 3,4-trans-3-[2-(Carbamoylmethoxy)phenyl]-2-[2-(4-methylphenyl)ethyl]-1-oxo-1,2,3,4-tetrahydro-4-isochinolincarbonsäure

Ausgehend von 2-(4-Methylphenyl)ethylamin [R = 2-(4-methylphenyl)ethyl]:
LC-MS (Methode B): Rₜ = 2.59 min
MS (ESIpos): m/z = 459 (M+H)⁺.

### Beispiel IX

### 3,4-trans-3-[2-(Carbamoylmethoxy)phenyl]-2-[2-(2-methoxyphenyl)ethyl]-1-oxo-1,2,3,4-tetrahydro-4-isochinolincarbonsäure

Ausgehend von 2-(2-Methoxyphenyl)ethylamin [R = 2-(2-methoxyphenyl)ethyl]:
LC-MS (Methode B): Rₜ = 2.50 min
MS (ESIpos): m/z = 475 (M+H)⁺.

### Beispiel X

### 3,4-trans-3-[2-(Carbamoylmethoxy)phenyl]-1-oxo-1,2,3,4-tetrahydro-2-(3,3,3-trifluorpropyl)-4-isochinolincarbonsäure

Ausgehend von 3,3,3-Trifluorpropylamin [R = 3,3,3-trifluorpropyl]:
LC-MS (Methode B): Rₜ = 2.37 min
MS (ESIpos): m/z = 437 (M+H)⁺.

### Beispiel XI

### 3,4-trans-2-n-Butyl-3-[2-(carbamoylmethoxy)phenyl]-1-oxo-1,2,3,4-tetrahydro-4-isochinolincarbonsäure

Ausgehend von n-Butylamin unter Zusatz von Toluol zum Orthoameisensäuretrimethylester im ersten Reaktionsschritt [R = n-butyl]:
LC-MS (Methode A): Rₜ = 3.33 min
MS (ESIpos): m/z = 397 (M+H)⁺.

### Beispiel XII

### 3,4-trans-3-[2-(Carbamoylmethoxy)phenyl]-2-n-hexyl-1-oxo-1,2,3,4-tetrahydro-4-isochinolincarbonsäure

Ausgehend von n-Hexylamin unter Zusatz von Toluol zum Orthoameisensäuretrimethylester im ersten Reaktionsschritt [R = n-hexyl]:
LC-MS (Methode A): Rₜ = 3.76 min
MS (ESIpos): m/z = 425 (M+H)⁺.

Analog, jedoch in Dichlormethan als Lösungsmittel sowohl für den ersten wie auch den zweiten Reaktionsschritt, wurden erhalten:

### Beispiel XIII

### 3,4-trans-2-[2-(1-Cyclohexen-1-yl)ethyl]-3-{2-[2-(4-methyl-1-piperazinyl)-2-oxoethoxy]-phenyl}-1-oxo-1,2,3,4-tetrahydro-4-isochinolincarbonsäure

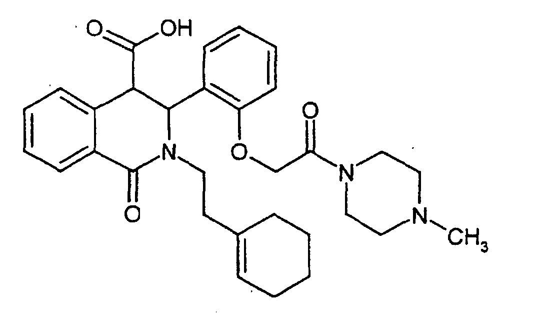

MS (ESIpos): 532 (M+H)⁺
HPLC: Rₜ = 4.11 min

### Beispiel XIV

### 3,4-trans-2-[2-(1-Cyclohexen-1-yl)ethyl]-3-{2-[2-(isopropylamino)-2-oxoethoxy]-phenyl}-1-oxo-1,2,3,4-tetrahydro-4-isochinolincarbonsäure

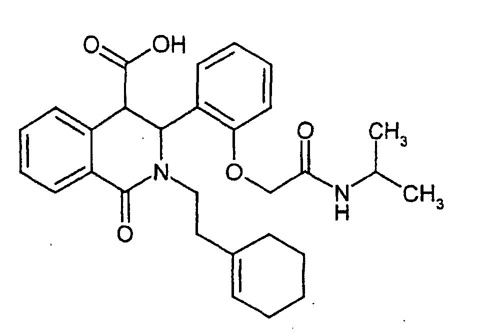

MS (ESIpos): 491 (M+H)⁺
HPLC: Rₜ = 4.58 min

Analog zu den beiden zuvor genannten Beispielen, jedoch in Toluol als Lösungsmittel für den ersten Reaktionsschritt, wurde hergestellt:

### Beispiel XV

### 3,4-traris-2-[2-(1-Cyclohexen-1-yl)ethyl]-1-oxo-3-{2-[2-oxo-2-(1-piperidinyl)-ethoxy]phenyl}-1,2,3,4-tetrahydro-4-isochinolincarbonsäure

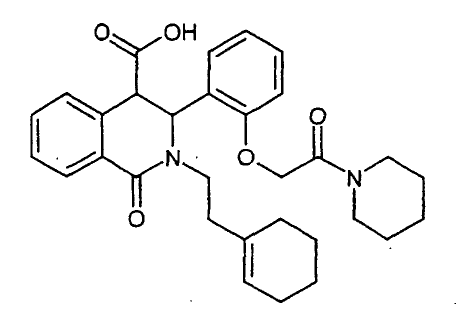

MS (ESIpos): 517 (M+H)⁺
¹H-NMR (MHz, DMSO-d₆): δ = 1.3-1.7 (m, 10H), 1.8-2.0 (m, 4H), 2.05-2.35 (m, 2H), 2.65-2.85 (m, 1H), 3.35-3.48 (br, 1H), 3.98 (m, 4H), 4.59 (s, br, 1H), 5.38 (s, br, 1H), 5.69 (s, br, 1H), 6.5-6.6 (m, 1H), 6.71 (t, 1H), 6.94 (d, 1H), 7.0-7.45 (m, 6H), 7.85-7.95 (m, 1H).

### Ausführungsbeispiele:

### Beispiel 1

### 3,4-trans-3-[2-(2-Carbamoylmethoxy)phenyl]-2-[2-(1-cyclohexen-1-yl)ethyl]-1-oxo-N-(3-pyridinylmethyl)-1,2,3,4-tetrahydro-4-isochinolincarbonsäureamid

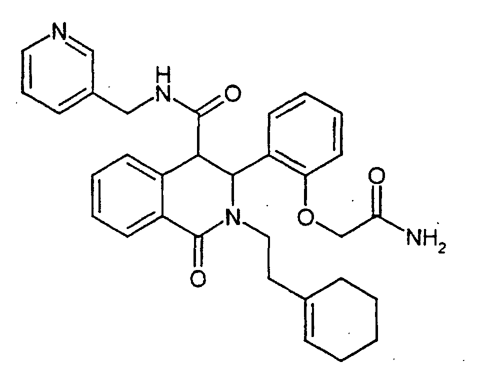

Zu einer eisgekühlten Lösung von 1,12 g (2,49 mmol) 3-[2-(2-Carbamoylmethoxy)-phenyl]-2-[2-(1-cyclohexen-1-yl)ethyl]-1-oxo-1,2,3,4-tetrahydro-4-isochinolincarbonsäure in wasserfreiem Dichlormethan werden unter Argon 297 mg (2,74 mmol) 3-Picolylamin, 542 mg (2,83 mmol) N-(3-Dimethylaminopropyl)-N'-ethylcarbodiimid-Hydrochlorid, 390 mg (2,89 mmol) 1-Hydroxy-1H-benzotriazol und 504 mg (4,99 mmol) Triethylamin gegeben. Man lässt die Reaktionsmischung über Nacht bei RT rühren. Dann wird der Ansatz mit Dichlormethan verdünnt, mit Wasser und gesättigter Natriumchloridlösung gewaschen und über Natriumsulfat getrocknet. Man engt im Vakuum ein und reinigt den verbleibenden Rückstand durch Chromatografie (HPLC, Acetonitril/Wasser 30:70 - 95:5). Man erhält so 1,04 g (78 %) der Zielverbindung als weißen Feststoff.
MS (DCI/NH₃): 539 (M+H)⁺
HPLC: Rₜ = 4.16 min
¹H-NMR (400 MHz, CDCl₃): δ = 1.50-1.59 (m, 4H); 1.93 (s, 4H); 2.12 (m, 2H); 2.70-2.77 (m, 1H); 4.04 (s, 1H); 4.17-4.24 (m, 1H); 4.28-4.33 (dd, 1H); 4.43-4.48 (dd, 1H); 4.51-4.66 (AB-System, 2H); 5.41 (s, 1H); 5.83 (s, 1H); 5.89-5.92 (t, 1H); 5.98 (s, 1H); 6.74-6.86 (m, 3H); 7.07 (d, 1H); 7.10-7.24 (m, 2H); 7.43 (m, 3H); 8.20-8.22 (m, 1H); 8.37 (m, 1H); 8.45 (m, 1H); 8.56 (s, 1H).

Das racemische Produkt lässt sich durch präparative Hochdruckflüssigkeits-chromatographie an chiralem Trägermaterial in die Enantiomere trennen (Säule: Daicel Chiralpak AS 10 µm, 250 x 20 mm, Eluent: 50 % iso-Hexan / 50 % Isopropanol, Flussrate: 13 ml/min, Injektionsvolumen: 500 µl, Temperatur: 45°C).
Enantiomer A: Rₜ = 15.09 min; [α]_{D} ^{20.0} (c = 0,50; CHCl₃) = - 18.9
Enantiomer B: Rₜ = 17.46 min; [α]_{D} ^{20.2} (c = 0,51; CHCl₃) = + 18.7
(Säule: Daicel Chiralpak AS 10 µm 250 x 4.6 mm, Eluent: 50 % iso-Hexan / 50 % Isopropanol, Flussrate: 0,5 ml/min, Injektionsvolumen: 5 µl, Temperatur: 42°C).

In analoger Weise wurden hergestellt:

### Beispiel 2

### 3,4-trans-3-[2-(2-Carbamoylmethoxy)phenyl]-2-[2-(1-cyclohexen-1-yl)ethyl]-1-oxo-N-(2-pyridinylmethyl)-1,2,3,4-tetrahydro-4-isochinolincarbonsäureamid

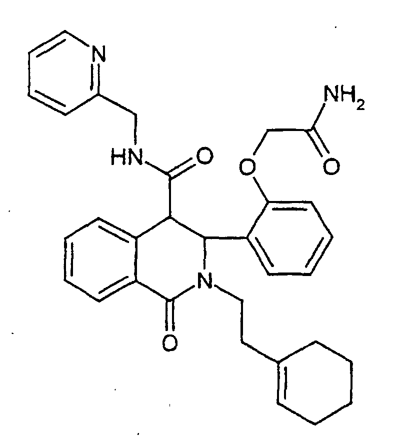

MS (ESI): 539 (M+H)⁺
¹H-NMR (300 MHz, DMSO): δ = 1.42-1.51 (m, 4H); 1.82-1.86 (m, 4H); 2.01-2.06 (m, 2H); 2.68-2.78 (m, 1H); 3.92-4.02 (m, 1H); 4.19 (s, 1H); 4.39 (d, 2H); 4.53-4.66 (AB-System, 2H); 5.29 (s, 1H); 5.62 (s, 1H); 6.60 (d, 1H); 6.79 (t, 1H); 6.99 (d, 1H); 7.15-7.25 (m, 4H); 7.39-7.44 (m, 2H); 7.60 (s, 1H); 7.67-7.73 (dt, 1H); 7.81 (s, 1H); 7.98-8.01 (m, 1H); 8.14 (t, 1H); 8.43 (d, 1H).

### Beispiel 3

### 3,4-trans-N-Benzyl-3-[2-(carbamoylmethoxy)phenyl]-2-[2-(1-cyclohexen-1-yl)-ethyl]-1-oxo-1,2,3,4-tetrahydro-4-isochinolincarbonsäureamid

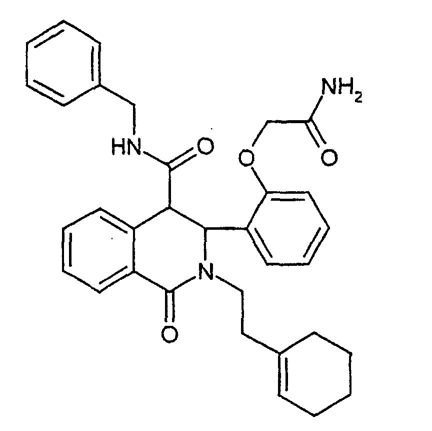

MS (ESI): 538 (M+H)⁺
¹H-NMR (300 MHz, DMSO): δ = 1.44-1.62 (m, 4H); 1.82-1.88 (m, 4H); 1.98-2.03 (m, 2H); 2.66-2.76 (m, 1H); 3.89-3.98 (m, 1H); 4.13 (s, 1H); 4.29 (d, 2H); 4.52-4.65 (AB-System, 2H); 5.29 (s, 1H); 5.56 (s, 1H); 6.59 (d, 1H); 6.75-6.80 (t, 1H); 6.98 (d, 1H); 7.18-7.29 (m, 7H); 7.39-7.41 (m, 2H); 7.62 (s, 1H); 7.83 (s, 1H); 7.95-7.99 (m, 1H); 8.13-8.18 (t, 1H).

### Beispiel 4

### 3,4-trans-2-n-Butyl-3-[2-(carbamoylmethoxy)phenyl]-1-oxo-N-(3-pyridinyl-methyl)-1,2,3,4-tetrahydro-4-isochinolincarbonsäureamid

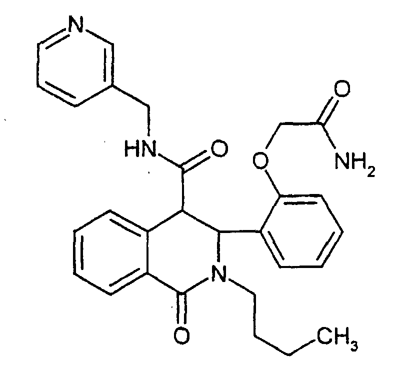

¹H-NMR (300 MHz, CDCl₃): δ = 0.91 (t, 3H); 1.24-1.34 (quin., 2H); 1.45-1.53 (m, 2H); 2.58-2.67 (quin., 1H); 4.04 (s, 1H); 4.09-4.23 (m, 1H); 4.27-4.34 (dd, 1H); 4.42-4.50 (dd, 1H); 4.52-4.69 (AB-System, 2H); 5.62 (t, 1H); 5.73 (s, 1H); 5.82 (s, 1H); 6.75-6.87 (m, 3H); 7.07-7.10 (m, 1H); 7.21-7.23 (m, 2H); 7.43 (m, 3H); 8.25-8.28 (m, 1H); 8.41 (d, 1H); 8.50 (dd, 1H); 8.55 (s, 1H).

### Beispiel 5

### 3,4-trans-3-[2-(Carbamoylmethoxy)phenyl]-1-oxo-2-(2-phenylethyl)-N-(3-pyridinylmethyl)-1,2,3,4-tetrahydro-4-isochinolincarbonsäureamid

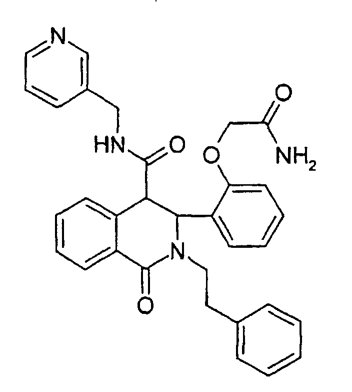

MS (ESI): 535 (M+H)⁺
¹H-NMR (200 MHz, CDCl₃): δ = 2.71-2.99 (m, 3H); 4.06 (s, 1H); 4.21-4.46 (m, 3H); 4.50-4.71 (AB-System, 2H); 5.63 (t, 1H); 5.75 (s, 1H); 5.91 (s, 1H); 6.77-6.87 (m, 3H); 7.07-7.24 (m, 8H); 7.41-7.47 (m, 3H); 8.25-8.30 (m, 1H); 8.40 (d, 1H); 8.46 (dd, 1.5Hz; 1H); 8.53 (s, 1H).

### Beispiel 6

### 3,4-trans-3-[2-(Carbamoylmethoxy)phenyl]-2-[2-(2-methoxyphenyl)ethyl]-1-oxo-N-(3-pyridinylmethyl)-1,2,3,4-tetrahydro-4-isochinolincarbonsäureamid

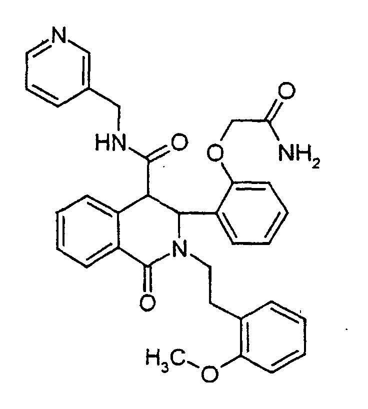

MS (ESI): 565 (M+H)⁺
¹H-NMR (200 MHz, CDCl₃): δ = 2.81-2.87 (m, 3H); 3.79 (s, 3H); 4.05 (s, 1H); 4.24-4.49 (m, 3H), 4.51-4.73 (AB-System, J_{AB}=14.5Hz, 2H); 5.61 (t, J=6Hz, 1H); 5.76 (s, 1H); 5.99 (s, 1H); 6.77-6.88 (m, 5H); 7.07-7.21 (m, 5H); 7.44-7.50 (m, 3H); 8.26-8.30 (m, 1H); 8.40 (d, J=2Hz, 1H); 8.47 (dd, J=5Hz, 1.5Hz, 1H); 8.68 (s, 1H).

### Beispiel 7

### 3,4-trans-3-[2-(Carbamoylmethoxy)phenyl]-2-n-hexyl-1-oxo-N-(3-pyridinyl-methyl)-1,2,3,4-tetrahydro-4-isochinolincarbonsäureamid

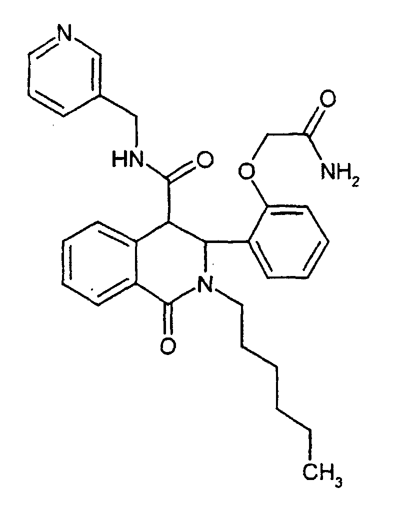

MS (ESI): 515 (M+H)⁺
¹H-NMR (200 MHz, CDCl₃): δ = 0.84-0.90 (t, J=6.5Hz, 3H); 1.27 (m, 6H); 1.49-1.52 (m, 2H); 2.56-2.70 (m, 1H); 4.04 (s, 1H); 4.09-4.53 (m, 3H); 4.50-4.70 (AB-System, J_{AB}=14.5Hz, 2H); 5.60 (t, J=6Hz, 1H); 5.73 (s, 1H); 5.82 (s, 1H); 6.74-6.87 (m, 3H); 7.06-7.10 (m, 1H); 7.20-7.22 (m, 2H); 7.42-7.50 (m, 3H); 8.24-8.29 (m, 1H); 8.40 (d, J=1.5Hz, 1H); 8.50 (dd, J=5Hz, 1.5Hz, 1H); 8.55 (s, 1H).

### Beispiel 8

### 3,4-trans-3-[2-(Carbamoylmethoxy)phenyl]-2-[2-(3,4-dimethoxyphenyl)ethyl]-1-oxo-N-(3-pyridinylmethyl)-1,2,3,4-tetrahydro-4-isochinolincarbonsäureamid

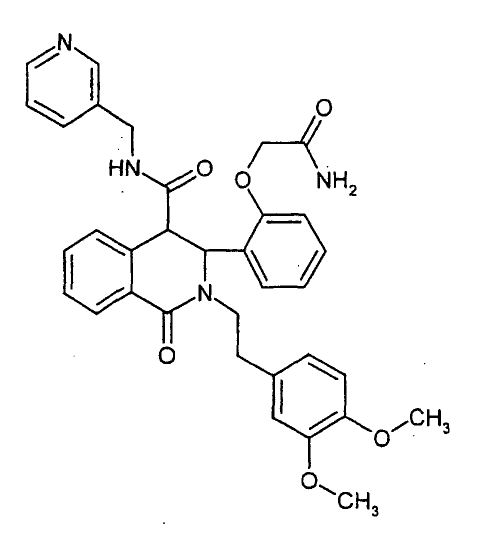

MS (ESI): 595 (M+H)⁺
¹H-NMR (200 MHz, CDCl₃): δ = 2.68-3.00 (m, 3H); 3.83 (s, 3H); 3.87 (s, 3H); 4.06 (s, 1H); 4.23-4.48 (m, 3H); 4.50-4.71 (AB-System, J_{AB}=14.5Hz, 2H); 5.63 (t, J=6Hz, 1H); 5.75 (s, 1H); 5.90 (s, 1H); 6.75-6.87 (m, 6H); 7.08-7.12 (m, 1H); 7.17-7.21 (m, 2H); 7.41-7.49 (m, 3H); 8.26-8.30 (m, 1H); 8.41 (d, J=1.5Hz, 1H); 8.47 (dd, J=5Hz, 1.5Hz, 1H); 8.53 (s, 1H).

### Beispiel 9

### 3,4-trans-3-[2-(Carbamoylmethoxy)phenyl]-2-[2-(4-methylphenyl)ethyl]-1-oxo-N-(3-pyridinylmethyl)-1,2,3,4-tetrahydro-4-isochinolincarbonsäureamid

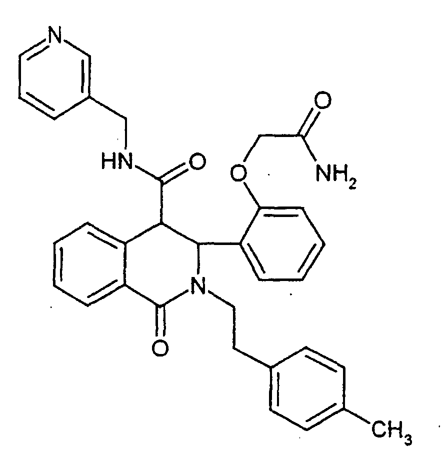

MS (ESI): 549 (M+H)⁺
¹H-NMR (200 MHz, CDCl₃): δ = 2.29 (s, 3H); 2.66-2.96 (m, 3H); 4.05 (s, 1H); 4.22-4.46 (m, 3H); 4.50-4.71 (AB-System, J_{AB}=14.5Hz, 2H); 5.62 (t, J=6Hz, 1H); 5.75 (s, 1H); 5.90 (s, 1H); 6.74-6.87 (m, 3H); 7.04-7.10 (m, 5H); 7.14-7.21 (m, 2H); 7.41-7.48 (m, 3H); 8.25-8.29 (m, 1H); 8.40 (d, J=2Hz, 1H); 8.46 (dd, J=5Hz, 1.5Hz, 1H); 8.54 (s, 1H).

### Beispiel 10

### 3,4-trans-3-[2-(Carbamoylmethoxy)phenyl]-1-oxo-N-(3-pyridinylmethyl)-1,2,3,4-tetrahydro-2-(3,3,3-trifluorpropyl)-4-isochinolincarbonsäureamid

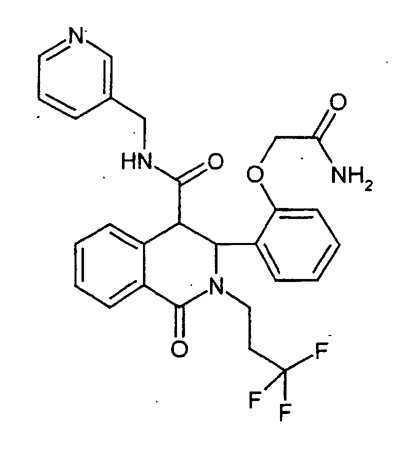

MS (ESI): 527 (M+H)⁺
¹H-NMR (200 MHz, CDCl₃): δ = 2.26-2.46 (m, 2H); 2.82-2.96 (quin., J=7Hz, 1H); 4.04 (s, 1H); 4.21-4.54 (m, 3H); 4.51-4.70 (AB-System, J_{AB}=14.5Hz, 2H); 5.66 (t, J=6Hz, 1H); 5.74 (s, 1H); 5.84 (s, 1H); 6.70-6.89 (m, 3H); 7.09-7.13 (m, 1H); 7.21 (m, 2H); 7.43-7.52 (m, 3H); 8.24-8.28 (m, 1H); 8.40 (d, J=2Hz, 2H); 8.51 (m, 1H).

### Beispiel 11

### 3,4-trans-2-[2-(1-Cyclohexen-1-yl)ethyl]-1-oxo-3-{2-[2-oxo-2-(1-piperidinyl)-ethoxy]phenyl}-N-(2-pyridinylmethyl)-1,2,3,4-tetrahydro-4-isochinolincarbonsäureamid

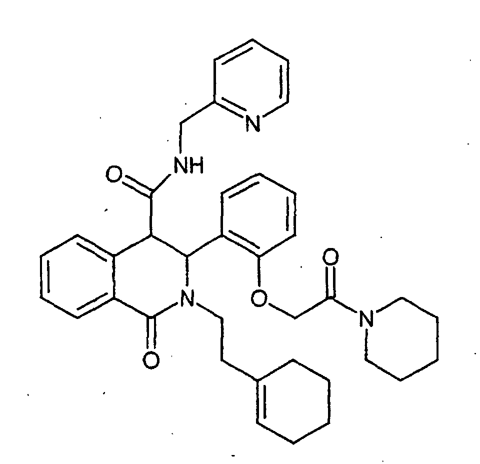

MS (ESI): 607 (M+H)⁺
¹H-NMR (300 MHz, DMSO): δ = 1.43-1.57 (m, 10H); 1.77-1.91 (m, 4H); 1.96-2.09 (m, 2H); 2.57-2.75 (m, 1H); 3.40-3.51 (m, 4H); 3.90-3.99 (m, 1H); 4.28 (s, 1H); 4.28-4.51 (m, 2H); 4.94-5.05 (AB-System, J_{AB}=14.5Hz, 2H); 5.30 (s, 1H); 5.47 (s, 1H); 6.59 (d, J=7.5Hz, 1H); 6.75-6.80 (m, 1H); 7.11-7.39 (m, 7H); 7.68-7.74 (dt, J=7.5Hz, 1.5Hz, 1H); 7.93-7.96 (m, 1H); 8.47 (d, J=4Hz, 1H); 9.02-9.06 (t, J=6Hz, 1H).

### Beispiel 12

### 3,4-trans-2-[2-(1-Cyclohexen-1-yl)ethyl]-1-oxo-3-{2-[2-oxo-2-(1-piperidinyl)-ethoxy]phenyl}-N-(3-pyridinylmethyl)-1,2,3,4-tetrahydro-4-isochinolincarbonsäureamid

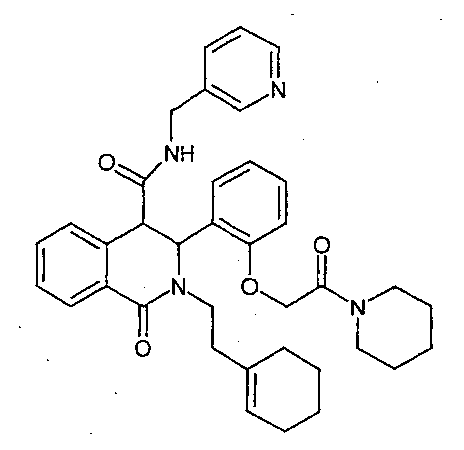

MS (ESI): 607 (M+H)⁺
¹H-NMR (300 MHz, DMSO): δ = 1.49-1.59 (m, 10H); 1.78-1.88 (m, 6H); 2.58-2.63 (m, 1H); 3.46 (m, 4H); 3.86-4.02 (m, 1H); 4.20 (s, 1H); 4.37 (m, 2H); 4.90-5.06 (m, 2H); 5.26 (s, br, 1H); 5.33 (s, 1H); 6.56 (d, J=7.5Hz, 1H); 6.73-6.79 (m, 1H); 7.05-7.09 (m, 1H); 7.14- 7.19 (m, 2H); 7.28-7.38 (m, 3H); 7.69-7.73 (m, 1H); 7.91-7.96 (m, 1H); 8.43 (dd, J=4.5Hz, 1.5Hz, 1H); 8.54 (d, J=1.5Hz, 1H); 9.19-9.25 (t, J=6Hz, 1H).

### Beispiel 13

### 3,4-trans-2-[2-(1-Cyclohexen-1-yl)ethyl]-3-{2-[2-(4-methyl-1-piperazinyl)-2-oxoethoxy]phenyl}-1-oxo-N-(3-pyridinylmethyl)-1,2,3,4-tetrahydro-4-isochinolincarbonsäureamid

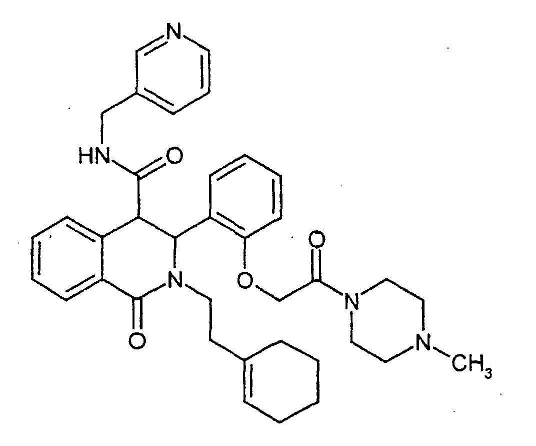

MS (ESI): 622 (M+H)⁺
¹H-NMR (300 MHz, DMSO): δ = 1.43-1.51 (m, 4H); 1.78-1.94 (m, 6H); 2.21 (s, 3H); 2.31-2.37 (m, 4H); 2.53-2.64 (m, 1H); 3.50 (m, 4H); 3.88-3.97 (m, 1H); 4.19 (s, 1H); 4.30-4.44 (m, 2H); 4.94-5.05 (AB-System, J_{AB}=15Hz, 2H); 5.26 (s, 1H); 5.33 (s, 1H); 6.56 (d, J=7.5Hz, 1H); 6.74-6.79 (m, 1H); 7.05-7.09 (m, 1H); 7.13- 7.22 (m, 2H); 7.29-7.37 (m, 3H); 7.69 (m, 1H); 7.94 (m, 1H); 8.43 (dd, J=4.5Hz, 1.5Hz, 1H); 8.53 (d, J=1.5Hz, 1H); 9.08-9.12 (t, J=6Hz, 1H).

### Beispiel 14

### 3,4-trans-2-[2-(1-Cyclohexen-1-yl)ethyl]-3-{2-[2-(isopropylamino)-2-oxoethoxy]-phenyl}-1-oxo-N-(2-pyridinylmethyl)-1,2,3,4-tetrahydro-4-isochinolincarbonsäureamid

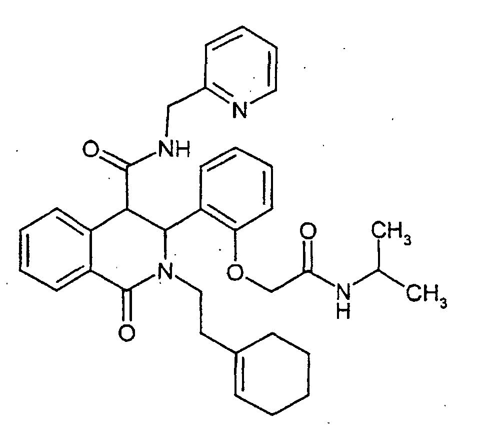

MS (ESI): 581 (M+H)⁺
¹H-NMR (200 MHz, DMSO): δ = 1.01 (d, J=6.5Hz, 3H); 1.12 (d, J=6.5Hz, 3H); 1.41-1.57 (m, 4H); 1.77-1.91 (m, 4H); 2.01-2.09 (m, 2H); 2.58-2.72 (m, 1H); 3.17 (d, J=5Hz, 1H); 3.93-4.11 (m, 2H); 4.15 (s, 1H); 4.28-4.47 (m, 2H); 4.50-4.70 (AB-System, J_{AB}=14Hz, 2H); 5.30 (s, b, 1H); 5.67 (s, 1H); 6.60 (m, 1H); 6.76-6.83 (t, J=7.5Hz, 1H); 6.99 (d, J=8Hz, 1H); 7.15-7.29 (m, 3H); 7.43-7.47 (t, J=4Hz, 2H); 7.66-7.74 (dt, J=7.5Hz, 1.5Hz, 1H); 7.98-8.18 (m, 3H); 8.43 (d, J=5Hz, 1H).

### Beispiel 15

### 3,4-trans-2-[2-(1-Cyclohexen-1-yl)ethyl]-1-oxo-3-{2-[2-oxo-2-(1-piperidinyl)-ethoxy]phenyl}-N-(4-pyridinylmethyl)-1,2,3,4-tetrahydro-4-isochinolincarbonsäureamid

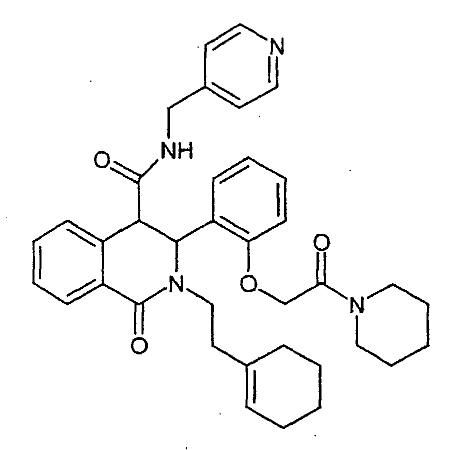

MS (ESIpos): m/z = 607 (M+H)⁺
¹H-NMR (300 MHz, DMSO-d₆): δ = 1.39-1.65 (m, 10H), 1.74-2.03 (m, 6H), 2.58-2.74 (m, 1H), 3.45 (d, 4H), 3.89-4.00 (m, 1H), 4.24 (s, 1H), 4.30-4.45 (m, 2H), 4.93-5.06 (m, 2H), 5.27 (s, 1H), 5.41 (s, 1H), 6.57 (d, 1H), 6.77 (dt, 1H), 7.09-7.22 (m, 3H), 7.29 (d, 2H), 7.34-7.39 (m, 2H), 7.91-7.97 (m, 1H), 8.47 (d, 2H), 9.17 (t, 1H).

### Beispiel 16

### 3,4-trans-2-[2-(1-Cyclohexen-1-yl)ethyl]-3-{2-[2-(isopropylamino)-2-oxoethoxy]-phenyl}-1-oxo-N-(3-pyridinylmethyl)-1,2,3,4-tetrahydro-4-isochinolincarbonsäureamid

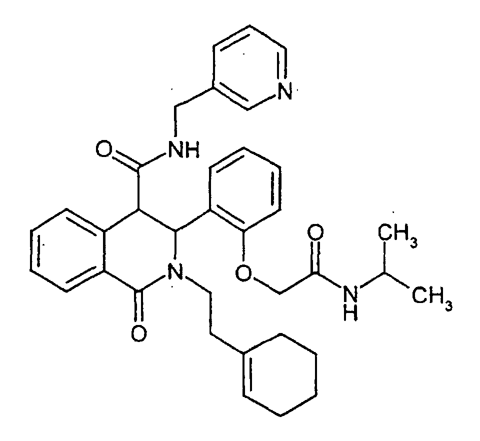

MS (ESIpos): m/z = 5 81 (M+H)⁺
¹H-NMR (300 MHz, DMSO-d₆): δ = 1.07 (d, 3H), 1.16 (d, 3H), 1.40-1.55 (m, 4H), 1.78-2.0 (m, 6H), 2.52-2.64 (m, 1H), 3.94-4.11 (m, 3H), 4.23-4.39 (m, 2H), 4.63 (quart., 1H), 5.28 (s, 1H), 5.56 (s, 1H), 5.76 (s, 1H), 6.57 (d, 1H), 6.78 (t, 1H), 6.98 (d, 1H), 7.17-7.23 (m, 2H), 7.27-7.31 (m, 1H), 7.40-7.45 (m, 2H), 7.58-7.61 (m, 1H), 7.97-8.00 (m, 1H), 8.19-8.25 (m, 2H), 8.40-8.46 (m, 2H).

HPLC-Methode:
Säule: Kromasil C 18 60*2;
Injektionsvolumen 1,00 µl;
Fluss: 0,75 ml/min;
Eluent: A = 5 ml HClO₄/l H₂O,
   B = CH₃CN;
Gradient [t (min): A/B]: 0,5: 98/2; 4,5: 10/90; 6,5: 10/90; 6,7: 98/2; 7,5: 98/2.

LC-MS-Methode A:
Säule: Symmetry C18;
Injektionsvolumen: 5 µl;
Fluss: 0,5 ml/min;
Eluent: A = CH₃CN + 0,1% Ameisensäure,
   B = H₂O + 0,1 % Ameisensäure;
Gradient [t (min): A/B]: 0,0: 10/90; 4,0: 90/10; 6,0: 90/10; 6,1: 10/90 (Fluss 1,0 ml/min); 7,5: 10/90.
MS: ESI.

LC-MS-Methode B:
Säule: Symmetry C18;
Injektionsvolumen: 2 µl;
Fluss: 0,9 ml/min;
Eluent: A = CH₃CN,
   B = 0,3 g 30%ige HCl/l H₂O;
Gradient [t (min): A/B]: 0,0: 10/90; 3,0: 90/10 (Fluss: 1,2 ml/min); 6,0: 90/10 (Fluss: 1,2 ml/min).
MS: ESI.

## Patentansprüche

1. Verbindungen der allgemeinen Formel (I), 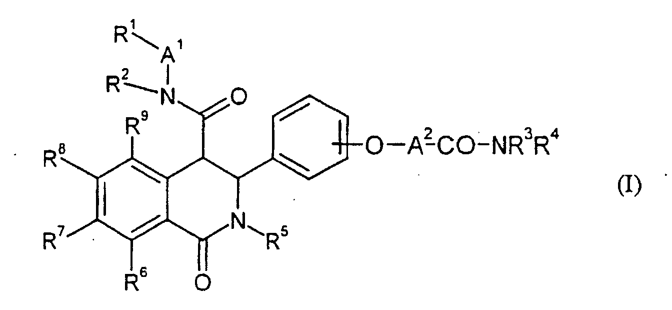 in welcher
R¹ für Phenyl oder 5- bis 6-gliedriges Heteroaryl steht, wobei gegebenenfalls Phenyl und Heteroaryl gleich oder verschieden durch Reste ausgewählt aus der Gruppe Halogen, Formyl, Carbamoyl, Cyan, Hydroxy, Trifluormethyl, Trifluormethoxy, Nitro, (C₁-C₆)-Alkyl, (C₁-C₆)-Alkoxy und (C₁-C₆)-Alkylthio substituiert sind,
A¹ für eine Bindung oder (C₁-C₆)-Alkandiyl steht,
R² für Wasserstoff oder (C₁-C₆)-Alkyl steht,
R³ und R⁴ gleich oder verschieden sind und für Wasserstoff, (C₁-C₈)-Alkyl oder (C₃-C₈)-Cycloalkyl stehen,
oder
R³ und R⁴ gemeinsam mit dem benachbarten Stickstoffatom für einen Rest ausgewählt aus der Gruppe Pyrrolidyl, Piperidyl, Azepinyl und Piperazinyl stehen, wobei die Reste gegebenenfalls durch Methyl oder Ethyl substiuiert sind,
A² für (C₁-C₆)-Alkandiyl steht,
R⁵ für (C₁-C₈)-Alkyl, das gegebenenfalls durch einen Rest ausgewählt aus der Gruppe Trifluormethyl, Halogen, gegebenenfalls teilweise ungesättigtes (C₃-C₈)-Cycloalkyl und (C₆-C₁₀)-Aryl substituiert ist, steht, wobei Aryl seinerseits gegebenenfalls gleich oder verschieden durch Reste ausgewählt aus der Gruppe Halogen, Formyl, Carbamoyl, Cyan, Hydroxy, Trifluormethyl, Trifluormethoxy, Nitro, (C₁-C₆)-Alkyl, (C₁-C₆)-Alkoxy und (C₁-C₆)-Alkylthio substituiert ist,
und
R⁶, R⁷, R⁸ und R⁹ gleich oder verschieden sind und für Wasserstoff, Halogen, Formyl, Carbamoyl, Cyan, Hydroxy, Trifluormethyl, Trifluormethoxy, Nitro, (C₁-C₆)-Alkyl, (C₁-C₆)-Alkoxy oder (C₁-C₆)-Alkylthio stehen,
und deren Salze, Hydrate und/oder Solvate.

2. Verbindungen nach Anspruch 1, wobei
R¹ für 3-Pyridyl, das gegebenenfalls gleich oder verschieden durch Chlor, Fluor oder Methyl substituiert ist, steht,
A¹ für Methylen steht,
R² für Wasserstoff steht,
R³ und R⁴ für Wasserstoff stehen,
A² für Methylen steht,
R⁵ für (C₁-C₃)-Alkyl, das durch einen Rest ausgewählt aus der Gruppe Trifluormethyl, gegebenenfalls einfach ungesättigtes (C₅-C₇)-Cyclo0 alkyl und gegebenenfalls durch Methyl, Halogen oder Methoxy substituiertes Phenyl substituiert ist, steht, oder
für (C₄-C₆)-Alkyl, das gegebenenfalls durch Trifluormethyl substituiert ist, steht,
und
R⁶, R⁷, R⁸ und R⁹ für Wasserstoff stehen,
und deren Salze, Hydrate und/oder Solvate.

3. Verfahren zur Herstellung von Verbindungen der allgemeinen Formel (I) nach Anspruch 1, **dadurch gekennzeichnet, dass** man Verbindungen der allgemeinen Formel (VI) 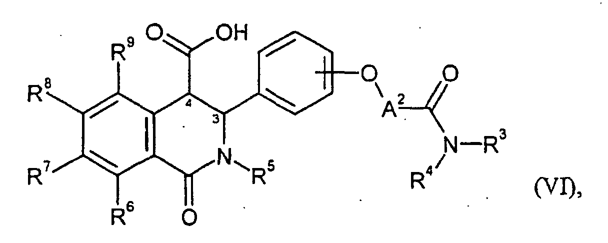 in welcher
A², R³, R⁴, R⁵, R⁶, R⁷, R⁸ und R⁹ die in Anspruch 1 angegebene Bedeutung haben,
mit Verbindungen der allgemeinen Formel (VII)
R¹-A¹-NH-R² (Vii),
in welcher
A¹, R¹ und R² die in Anspruch 1 angegebene Bedeutung haben,
umsetzt.

4. Verbindungen nach Anspruch 1 oder 2 zur Behandlung und/oder Prophylaxe von Krankheiten.

5. Arzneimittel enthaltend mindestens eine der Verbindungen nach Anspruch 1 oder 2 in Zusammenmischung mit mindestens einem pharmazeutisch verträglichen, im wesentlichen nichtgiftigen Träger oder Exzipienten.

6. Verwendung von Verbindungen nach Anspruch 1 oder 2 zur Herstellung eines Arzneimittels zur Behandlung und/oder Prophylaxe von Schmerzzuständen.

7. Arzneimittel nach Anspruch 5 zur Behandlung und/oder Prophylaxe von Schmerzzuständen.

## Claims

1. Compounds of the general formula (I) 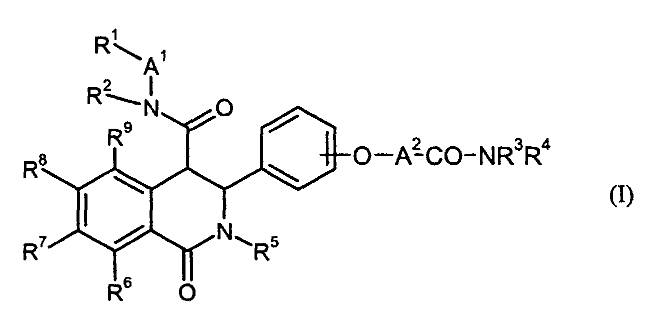 in which
R¹ is phenyl or 5- or 6-membered heteroaryl, where phenyl and heteroaryl may optionally be identically or differently substituted by radicals selected from the group of halogen, formyl, carbamoyl, cyano, hydroxyl, trifluoromethyl, trifluoromethoxy, nitro, (C₁-C₆)alkyl, (C₁-C₆)alkoxy and (C₁-C₆)alkylthio,
A¹ is a bond or (C₁-C₆)alkanediyl,
R² is hydrogen or (C₁-C₆)alkyl,
R³ and R⁴ are the same or different and are each hydrogen, (C₁-C₈)alkyl or (C₃-C₈)cycloalkyl,
or
R³ and R⁴ together with the adjacent nitrogen atom are a radical selected from the group of pyrrolidyl, piperidyl, azepinyl and piperazinyl, where the radicals may optionally be substituted by methyl or ethyl,
A² is (C₁-C₆)alkanediyl,
R⁵ is (C₁-C₈)alkyl which may optionally be substituted by a radical selected from the group of trifluoromethyl, halogen, saturated or partly unsaturated (C₃-C₈)cycloalkyl and (C₆-C₁₀)aryl, where aryl may itself optionally be substituted identically or differently by radicals selected from the group of halogen, formyl, carbamoyl, cyano, hydroxyl, trifluoromethyl, trifluoromethoxy, nitro, (C₁-C₆)alkyl, (C₁-C₆)alkoxy and (C₁-C₆)alkylthio,
and
R⁶, R⁷, R⁸ and R⁹ are the same or different and are each hydrogen, halogen, formyl, carbamoyl, cyano, hydroxyl, trifluoromethyl, trifluoromethoxy, nitro, (C₁-C₆)alkyl, (C₁-C₆)alkoxy or (C₁-C₆)alkylthio,
and their salts, hydrates and/or solvates.

2. Compounds according to Claim I, **characterized in that**
R¹ is 3-pyridyl which is optionally identically or differently substituted by chlorine, fluorine or methyl,
A¹ is methylene,
R² is hydrogen,
R³ and R⁴ are each hydrogen,
A² is methylene,
R⁵ is (C₁-C₃)alkyl which is substituted by a radical selected from the group of trifluoromethyl, unsaturated or monounsaturated (C₅-C₇)cycloalkyl and optionally methyl-, halogen- or methoxy-substituted phenyl, or
is (C₄-C₆)alkyl which is optionally substituted by trifluoromethyl, and
R⁶, R⁷, R⁸ and R⁹ are each hydrogen,
and their salts, hydrates and/or solvates.

3. Process for preparing compounds of the general formula (I) according to Claim 1, **characterized in that** compounds of the general formula (VI) 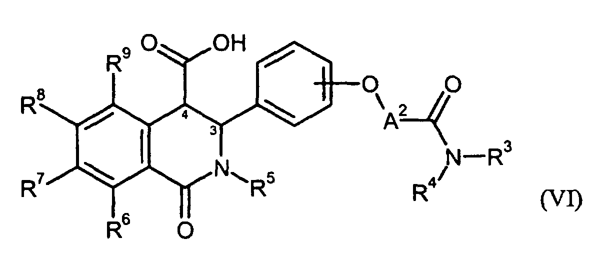 in which
A², R³, R⁴, R⁵, R⁶, R⁷, R⁸ and R⁹ are each as defined in Claim 1
are reacted with compounds of the general formula (VII)
R¹-A¹-NH-R² (VII)
in which
A¹, R¹ and R² are each as defined in Claim 1.

4. Compounds according to Claim 1 or 2 for the treatment and/or prophylaxis of diseases.

5. Pharmaceutical comprising at least one of the compounds according to Claim 1 or 2 in admixture with at least one pharmaceutically acceptable, substantially nontoxic carrier or excipient.

6. Use of compounds according to Claim 1 or 2 for producing a pharmaceutical for the treatment and/or prophylaxis of states of pain.

7. Pharmaceutical according to Claim 5 for the treatment and/or prophylaxis of states of pain.

## Revendications

1. Composés de formule générale (I) 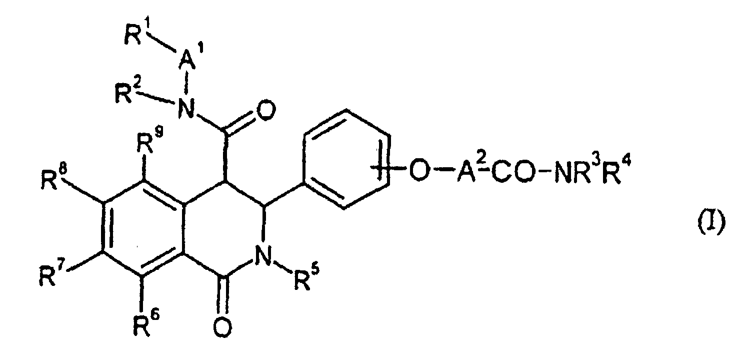 dans laquelle
R¹ représente un groupe phényle ou un groupe hétéroaryle pentagonal ou hexagonal, les restes phényle et hétéroaryle étant éventuellement substitués de façon identique ou différente par des restes choisis dans le groupe halogéno, formyle, carbamoyle, cyano, hydroxy, trifluorométhyle, trifluorométhoxy, nitro, alkyle en C₁ à C₆, alkoxy en C₁ à C₆ et alkylthio en C₁ à C₆,
A¹ représente une liaison ou un reste alcanediyle en C₁ à C₆,
R² représente l'hydrogène ou un reste alkyle en C₁ à C₆,
R³ et R⁴ sont identiques ou différents et représentent l'hydrogène, un reste alkyle en C₁ à C₈ ou cycloalkyle en C₃ à C₈,
ou bien
R³ et R⁴ forment, conjointement avec l'atome d'azote contigu, un reste choisi dans le groupe pyrrolidyle, pipéridyle, azépinyle et pipérazinyle, les restes étant éventuellement substitués par un radical méthyle ou éthyle,
A² est un reste alcanediyle en C₁ à C₆,
R⁵ est un reste alkyle en C₁ à C₈, qui est éventuellement substitué par un reste choisi dans le groupe trifluorométhyle, halogéno, cycloalkyle en C₃ à C₈, le cas échéant partiellement insaturé, et aryle en C₆ à C₁₀, le reste aryle pouvant lui-même être substitué de façon identique ou différente par des restes choisis dans le groupe halogéno, formyle, carbamoyle, cyano, hydroxy, trifluorométhyle, trifluorométhoxy, nitro, alkyle en C₁ à C₆, alkoxy en C₁ à C₆ et alkylthio en C₁ à C₆,
et
R⁶, R⁷, R⁸ et R⁹ sont identiques ou différents et représentent l'hydrogène, un halogène, un groupe formyle, carbamoyle, cyano, hydroxy, trifluorométhyle, trifluorométhoxy, nitro, alkyle en C₁ à C₆, alkoxy en C₁ à C₆ ou alkylthio en C₁ à C₆,
et leurs sels, leurs hydrates et/ou leurs produits de solvatation.

2. Composés suivant la revendication 1, dans lesquels
R¹ est un reste 3-pyridyle qui est éventuellement substitué de façon identique ou différente par du chlore, du fluor ou un radical méthyle,
A¹ est un reste méthylène,
R² représente l'hydrogène,
R³ et R⁴ représentent l'hydrogène,
A² est un reste méthylène,
R⁵ est un reste alkyle en C₁ à C₃ qui est éventuellement substitué par un reste choisi dans le groupe trifluorométhyle, cycloalkyle en C₅ à C₇ éventuellement mono-insaturé et phényle éventuellement substitué par un radical méthyle, halogéno ou méthoxy, ou bien
un reste alkyle en C₄ à C₆, qui est éventuellement substitué par un radical trifluorométhyle,
et
R⁶, R⁷, R⁸ et R⁹ représentent l'hydrogène,
et leurs sels, leurs hydrates et/ou leurs produits de solvatation.

3. Procédé de production de composés de formule générale (I) suivant la revendication 1, **caractérisé en ce qu'**on fait réagir des composés de formule générale (VI) 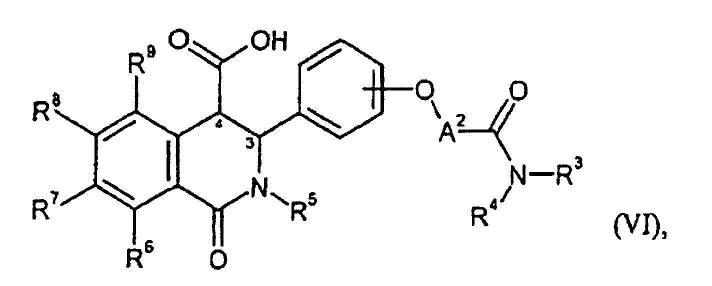 dans laquelle
A², R³, R⁴, R⁵, R⁶, R⁷, R⁸ et R⁹ ont la définition indiquée dans la revendication 1,
avec des composés de formule générale (VII)
**R**^{**1**}**-A**^{**1**}**-NH-R**^{**2**} **(VII),** (VII),
dans laquelle
A¹, R¹ et R² ont la définition indiquée dans la revendication 1.

4. Composés suivant la revendication 1 ou 2, destinés au traitement et/ou à la prophylaxie de maladies.

5. Médicament contenant au moins l'un des composés suivant la revendication 1 ou 2 en mélange avec au moins un support ou excipient principalement non toxique, acceptable du point de vue pharmaceutique.

6. Utilisation de composés suivant la revendication 1 ou 2 pour la préparation d'un médicament destiné au traitement et/ou à la prophylaxie d'états douloureux.

7. Médicament suivant la revendication 5, destiné au traitement et/ou à la prophylaxie d'états douloureux.
